# EUROPEAN PATENT APPLICATION

(11) **EP 4 008 404 A1**
(43) Date of publication of application: **08.06.2022**
(21) Application number: 20845661.6
(22) Date of filing: 31.07.2020
(51) Int. Cl.: A61P 35/00, A61P 43/00, C07K 14/705, C07K 14/725, C07K 16/28, C12N 5/0783, C12N 15/13, C12N 15/62, A61K 35/17

(54) **ANTIGEN RECEPTOR**

(30) Priority: 01.08.2019 JP 2019142357
(71) Applicant: Mie University, Tsu-shi, Mie 514-8507 (JP)
(72) Inventor: SHIKU, Hiroshi, Tsu-shi, Mie 514-8507 (JP); MIWA, Hiroshi, Tsu-shi, Mie 514-8507 (JP); AKAHORI, Yasushi, Tsu-shi, Mie 514-8507 (JP); FUJIWARA, Hiroshi, Tsu-shi, Mie 514-8507 (JP)
(74) Representative: Gevers & Orès
(86) International application number: PCT/JP2020/029437
(87) International publication number: WO 2021/020559

(57) **Abstract**

A novel antigen receptor is provided. An antigen receptor contains a GITRL domain at a position closer to the C-terminus via a self-cleaving peptide domain.

## Description

### Technical Field

The present invention relates to an antigen receptor and the like.

### Background Art

In addition to surgery, radiation therapy, and chemotherapy, immunotherapy has been attracting attention as the fourth treatment for cancer. Examples of immunotherapy currently available or approved to become available in the future in Japan include immune checkpoint inhibitors and chimeric antigen receptor (CAR) gene-modified T-cell therapy. CAR-T-cell therapy targeting CD19, which is expected to be effective against intractable B-cell acute lymphoblastic leukemia (B-ALL) and diffuse large B-cell lymphoma (DLBCL), has been approved in the United States, Europe, and other countries since 2017 and was approved in March 2019 in Japan. Although this therapy has a remarkable effect on intractable cases resistant to conventional treatment, including hematopoietic stem cell transplantation, there are not a few relapsed and refractory cases; thus, further improvement has been desired. CAR-T-cell therapy for solid tumors is also expected to be developed in the future.

In order to enhance the effect of CAR-T-cell therapy, studies are in progress on, for example, combined use with immune checkpoint inhibitors and improvement in metabolism in the tumor microenvironment. The report that CD19 CAR-T cells with a costimulatory molecule 4-1BB ligand bound to 0028-003ζ as an intracellular domain have excellent persistence and more potent antitumor effects (Non-patent Literature 1) is highly thoughtprovoking.

### Citation List

### Non-patent Literature

NPL 1: Zhao Z. et al. Cancer Cell 28(4): 415-428, 2015

### Summary of Invention

### Technical Problem

An object of the present invention is to provide a novel antigen receptor. Preferably, an object of the present invention is to provide an antigen receptor with a high therapeutic or preventive effect on cancer.

### Solution to Problem

The present inventors conducted extensive research in view of the problem above and found that the problem can be solved by an antigen receptor that contains a GITRL domain at a position closer to the C-terminus via a self-cleaving peptide domain. The inventors conducted further research on the basis of this finding and completed the present invention. Specifically, the present invention includes the following subject matter.

### Item 1.

An antigen receptor comprising a GITRL domain at a position closer to the C-terminus via a self-cleaving peptide domain.

### Item 2.

The antigen receptor according to Item 1, comprising a core domain containing a variable region of an antibody or T-cell receptor, a transmembrane domain, and an intracellular domain of TCR.

### Item 3.

The antigen receptor according to Item 2, comprising the GITRL domain at a position closer to the C-terminus of the core domain via a self-cleaving peptide domain.

### Item 4.

The antigen receptor according to Item 2 or 3, wherein the core domain further contains an intracellular domain of a co-stimulator.

### Item 5.

The antigen receptor according to Item 4, wherein the co-stimulator is CD28.

### Item 6.

The antigen receptor according to any one of Items 1 to 5, which is a chimeric antigen receptor or a T-cell receptor.

### Item 7.

The antigen receptor according to any one of Items 2 to 6, wherein the variable region is capable of binding to CD19.

### Item 8.

A polynucleotide encoding the antigen receptor of any one of Items 1 to 7.

### Item 9. A cell comprising the polynucleotide of Item 9.

### Item 10.

The cell according to Item 9, which is a αβ T cell, a γδ T cell, or an NK cell.

### Item 11.

A pharmaceutical composition comprising the cell of Item 10.

### Item 12.

The pharmaceutical composition according to Item 11, which is for use in the treatment or prevention of cancer.

### Advantageous Effects of Invention

The present invention provides a novel antigen receptor. The antigen receptor effectively treats or prevents cancer.

### Brief Description of Drawings

Fig. 1 schematically shows the structure of 28z CAR.
Fig. 2 shows the base sequence of CD19 28z CAR.
Fig. 3 shows the base sequence and amino acid sequence of CD19 28z GITRL CAR.
Fig. 4 shows the results of Test Example 3 (CAR expression in αβ cells when the intracellular domain is CD28). The proportion of CAR-expressing cells is shown as a percentage. NGMC indicates non-gene modified cells (the same applies to other figures).
Fig. 5 shows the results of Test Example 3 (CAR expression in γδ cells when the intracellular domain is CD28). The proportion of CAR-expressing cells is shown as a percentage.
Fig. 6 shows the results of Test Example 3 (CAR expression in αβ cells when the intracellular domain is GITR). The proportion of CAR-expressing cells is shown as a percentage.
Fig. 7 shows the results of Test Example 3 (CAR expression in γδ cells when the intracellular domain is GITR). The proportion of CAR-expressing cells is shown as a percentage.
Fig. 8 shows the results of Test Example 4 (IFN-γ expression in αβ cells when the intracellular domain is CD28). The proportion of CAR-expressing cells and the proportion of IFN-γ-producing cells in CAR-expressing cells are shown as a percentage.
Fig. 9 shows the results of Test Example 4 (IFN-γ expression in γδ cells when the intracellular domain is CD28). The proportion of CAR-expressing cells and the proportion of IFN-γ-producing cells in CAR-expressing cells are shown as a percentage.
Fig. 10 shows the results of Test Example 4 (IFN-γ expression in αβ cells when the intracellular domain is GITR). The proportion of CAR-expressing cells and the proportion of IFN-γ-producing cells in CAR-expressing cells are shown as a percentage.
Fig. 11 shows the results of Test Example 4 (IFN-γ expression in γδ cells when the intracellular domain is GITR). The proportion of CAR-expressing cells and the proportion of IFN-γ-producing cells in CAR-expressing cells are shown as a percentage.
Fig. 12 shows the summary of Test Examples 3 and 4 for αβ cells (CAR expression and IFN production).
Fig. 13 shows the summary of Test Examples 3 and 4 for γδ cells (CAR expression and IFN production).
Fig. 14 shows the average values of the ratio of the GITRL-bound form to the GITRL-unbound form (GITRL-bound form/GITRL-unbound form) in the results of the bottom row of each of Figs. 12 and 13.

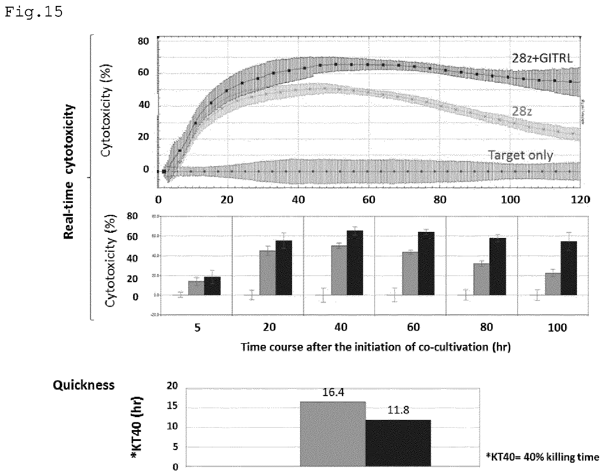

Fig. 15 shows the results of Test Example 5 (results of αβ cells). The top row shows the results of cytotoxicity recorded over time when each kind of effector cells was used. The middle row shows cytotoxicity at 5, 20, 40, 60, 80, 100, and 120 hours. The bottom row shows the time required to damage half of target cells. In the top and middle rows, the vertical axis represents cytotoxic activity (%) measured by xCELLigence, and the horizontal axis represents the elapsed time from addition of effector cells. The columns of each bar graph indicate, from the left side, the case in which effector cells were not allowed to act, the case in which effector cells into which CD19 28z was introduced were allowed to act, and the case in which effector cells into which CD19 28z-GITRL was introduced were allowed to act.

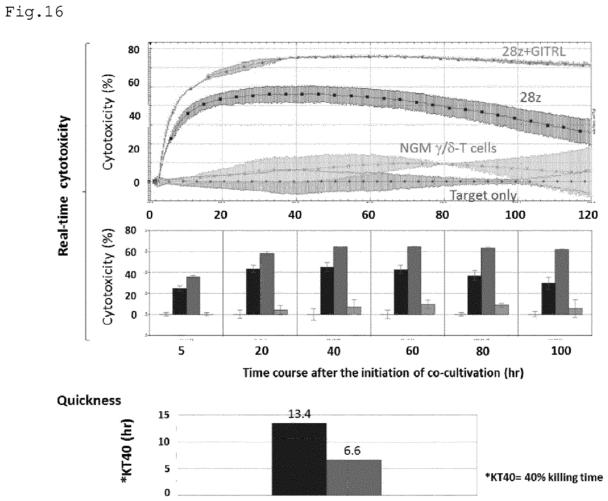

Fig. 16 shows the results of Test Example 5 (results of γδ cells). The top row shows the results of cytotoxicity recorded over time when each kind of effector cells was used. The middle row shows cytotoxicity at 5, 20, 40, 60, 80, 100, and 120 hours. The bottom row shows the time required to damage half of target cells. In the top and middle rows, the vertical axis represents cytotoxic activity (%) measured by xCELLigence, and the horizontal axis represents the elapsed time from addition of effector cells. The columns of each bar graph indicate, from the left side, the case in which effector cells were not allowed to act, the case in which effector cells into which CD19 28z was introduced were allowed to act, the case in which effector cells into which CD19 28z-GITRL was introduced were allowed to act, and the case in which NGM (non-gene modified) γδ cells were allowed to act.
Fig. 17 shows the results of Test Example 6. PBS indicates the case in which PBS was administered without administration of CAR-T cells, NGM-αβT indicates the case in which NGM (non-gene modified) T cells were administered, CD19 28z-αβT indicates the case in which T cells into which CD19 28z was introduced were administered, and CD19 28z-GITRL-αβT indicates the case in which T cells into which CD19 28z-GITRL was introduced were administered.
Fig. 18 shows the results of Test Example 7. The proportion of IFN-γ-positive cells in CD19CAR-positive cells is shown in each of the CD8 and CD4 fractions (framed in yellow, red lettering).
Fig. 19 shows the results of Test Example 8. The proportions of effector memory T cells and central memory T cells are shown in each of the TMRM low and TMRM high fractions.
Fig. 20 shows the results of Test Example 9. The vertical axis represents cytotoxic activity (%) measured by xCELLigence, and the horizontal axis represents the elapsed time from addition of effector cells.
Fig. 21 shows the results of Test Example 10. The vertical axis represents the abundance ratio of infused cells in peripheral blood.
Fig. 22 shows the results of Test Example 11. The results are analysis results of mouse peripheral blood 38 days after cell therapy, i.e., 10 days after the second NALM6 cell inoculation (NALM6/luc 5×10⁶ cells i.v./mouse, effector:target ratio = 1:1).
Fig. 23 shows the results of Test Example 12. These are analysis results of peripheral blood of 28z-GITRL CAR-T celltreated mouse #2 55 days after cell therapy, i.e., 27 days after the second inoculation of leukemia cells NALM6 to cause the mouse to bear a tumor.

### Description of Embodiments

In the present specification, the terms "comprising," "containing," and "including" include the concepts of comprising, containing, consisting essentially of, and consisting of.

### 1. Definition

In the present specification, the terms "comprising," "containing," and "including" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scorings schemes, Proc Natl Acad Sci USA. 87: 2264-2268(1990), Karlin S, Altschul SF. Applications and statistics for multiple high-scoring segments in molecular sequences, Proc Natl Acad Sci USA.90: 5873-7(1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/). The "identity" of base sequences is also defined in the same manner as above.

In the present specification, "conservative substitution" means the substitution of an amino acid residue with an amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

In the present specification, "CDR" is an abbreviation for complementarity determining region. CDR is a region in the variable regions of immunoglobulins or T-cell receptors and is deeply involved in the specific binding of an antibody or T-cell receptor to its antigen. The phrase "light-chain CDR" refers to a CDR present in the light-chain variable regions of immunoglobulins, and the phrase "heavy-chain CDR" refers to a CDR present in the heavy-chain variable regions of immunoglobulins. In T-cell receptors, there are also α chain, β chain, γ chain, δ chain, and like chains, and the same applies to the CDRs of these chains.

In the present specification, the phrase "variable region" refers to a region containing CDR1 to CDR3 (simply "CDRs 1-3" below). The order in which these CDRs 1-3 are arranged is not limited; however, the variable region preferably refers to a region in which CDR1, CDR2, and CDR3 are arranged in this order in the direction from the N-terminus toward the C-terminus or in the reverse order either consecutively or via other amino acid sequences referred to as "framework regions" (FRs), which are described later. The phrase "heavy-chain variable region" refers to a region of immunoglobulins in which heavy-chain CDRs 1-3 are arranged, and the phrase "light-chain variable region" refers to a region of immunoglobulins in which light-chain CDRs 1-3 are arranged. In T-cell receptors, there are also α chain, β chain, γ chain, δ chain, and like chains, and the same applies to the CDRs of these chains.

The regions other than CDRs 1-3 of each variable region are referred to as "framework regions" (FRs), as mentioned above. In particular, the region between the N-terminus and CDR1 of a variable region is defined as FR1, the region between CDR1 and CDR2 as FR2, the region between CDR2 and CDR3 as FR3, and the region between CDR3 and the C-terminus of a variable region as FR4.

### 2. Antigen Receptor

In an embodiment, the present invention relates to an antigen receptor containing a GITRL domain at a position closer to the C-terminus via a self-cleaving peptide domain (which may be referred to as "the antigen receptor of the present invention" in the present specification). The antigen receptor of the present invention is described below.

The antigen receptor can be any antigen receptor, and is, for example, a chimeric antigen receptor (CAR). The chimeric antigen receptor is typically a chimeric protein that has its single-chain antibody (scFv) composed of a light chain (VL) bound in tandem to a heavy chain (VH) of the variable region of a monoclonal antibody at a position closer to the N-terminus as a domain responsible for its capability of binding to an antigen and its T-cell receptor (TCR) ζ chain at a position closer to the C-terminus. T cells expressing CAR are referred to as "CAR-T cells."

The antigen receptor is not limited to a chimeric antigen receptor, and can be, for example, a T-cell receptor.

The antigen receptor of the present invention can be any antigen receptor that contains a GITRL domain at a position closer to the C-terminus via a self-cleaving peptide domain. This can increase the expression efficiency of the antigen receptor or the cytotoxic activity of T cells containing the antigen receptor. Expressing the antigen receptor of the present invention leads to cleavage of the self-cleaving peptide domain, allowing for GITRL in its free form to be intracellularly expressed.

In the present specification, the phrase "self-cleaving peptide" refers to a peptide sequence with cleavage activity occurring between two amino acid residues in the peptide sequence. Examples of self-cleaving peptides include 2A peptides and 2A-like peptides. For example, in 2A peptides or 2A-like peptides, cleavage occurs between the glycine residue and the proline residue of these peptides. This occurs because of the "ribosomal skipping mechanism," in which a normal peptide linkage between the glycine residue and the proline residue does not form during translation, and this does not affect the translation downstream. The ribosomal skipping mechanism is known in the art and is used in the expression of multiple proteins encoded by a single molecular messenger RNA (mRNA). The self-cleaving peptide for use in the present invention can be obtained from 2A peptides of viruses or 2A-like peptides that have equivalent functionality. For example, the self-cleaving peptide can be selected from the group consisting of 2A peptide derived from foot-and-mouth disease virus (FMDV) (F2A), 2A peptide derived from equine rhinitis A virus (ERAV) (E2A), 2A peptide derived from porcine teschovirus (PTV-1) (P2A), and 2A peptide derived from *Thosea asigna* virus (TaV) (T2A). The self-cleaving peptide domain may be mutated as long as the activity of the self-cleaving peptide domain is not greatly impaired.

The GITRL domain can be any domain containing the amino acid sequence of GITRL.

GITRL for use can be various types of GITRL derived from mammals, such as humans, monkeys, mice, rats, dogs, cats, and rabbits. Of these, GITRL derived from a target organism (e.g., humans) is preferable.

The amino acid sequences of GITRL derived from various organisms are known. Specifically, examples include proteins having the amino acid sequence represented by SEQ ID NO: 4. GITRL may also be one with the N-terminal signal peptide deleted.

GITRL may be mutated, for example, by the substitution, deletion, addition, or insertion of amino acids as long as GITRL maintains the capability of binding to its receptor, GITR. The mutation is preferably a substitution, and more preferably a conservative substitution from the standpoint of the lower likelihood of GITRL losing its capability of binding to GITR.

A preferable specific example of the amino acid sequence of GITRL is at least one member selected from the group consisting of the amino acid sequence described in the following (a) and the amino acid sequence described in the following (b):
(a) an amino acid sequence represented by any portion of SEQ ID NO: 4, and
(b) an amino acid sequence that has at least 85% identity with an amino acid sequence represented by any portion of SEQ ID NO: 4, and that constitutes a protein capable of binding to GITR.

In item (b) above, the identity is preferably at least 90%, more preferably at least 95%, still more preferably at least 98%, and particularly preferably at least 99%.

Whether GITRL is callable of binding to GITR can be determined in accordance with a known method or an equivalent method. The phrase "capable of binding to GITR" means that GITRL is capable of binding to GITR, for example, to an extent of at least 50%, at least 70%, at least 80%, at least 90%, or at least 95% of the binding capability of GITRL before mutation.

An example of the amino acid sequence described in item (b) above is (b') an amino acid sequence that has the substitution, deletion, addition, or insertion of one or multiple amino acids in a portion of the amino acid sequence represented by any portion of SEQ ID NO: 4, and that constitutes a protein capable of binding to GITR.

In item (b') above, "multiple amino acids" means, for example, 2 to 15 amino acids, preferably 2 to 10 amino acids, more preferably 2 to 5 amino acids, and more preferably 2 or 3 amino acids.

The antigen receptor of the present invention typically contains a domain responsible for the binding capability to its antigen (antigen-binding domain). The antigen is not particularly limited, and examples include various proteins or complexes that are expressed on the surface of cancer cells or T cells. Specific examples of antigens include CD19, GD2, GD3, CD20, CEA, HER2, EGFR, type III mutant EGFR, CD38, BCMA, MUC-1, PSMA, WT1, cancer testis antigens (e.g., NY-ESO-1 and MAGE-A4), mutation peptides (e.g., k-ras, h-ras, and p53), hTERT, PRAM, TYRP1, mesothelin, PMEL, mucin, and complexes of fragments of these antigens with MHC (pMHC). Of these, the antigen is preferably CD19 or GD2, and more preferably CD19. The antigen-binding domain typically contains one or more, preferably two, three, four, or five or more CDRs, or more preferably all of the six CDRs of an antibody or T-cell receptor for an antigen (e.g., in the case of CDRs of antibodies, heavy chain CDR1, heavy chain CDR2, heavy chain CDR3, light chain CDR1, light chain CDR2, and light chain CDR3). The antigen-binding domain more preferably contains a variable region of an antibody or T-cell receptor for an antigen (in the case of antibodies, for example, the heavy-chain variable regions and/or light-chain variable regions).

The antigen-binding domain preferably has the structure of scFv. The linker that links the heavy-chain variable region with the light-chain variable region can be any linker that maintains functionality of the antigen receptor. The linker is preferably a GS linker (typically, a linker having a repeated sequence containing GGGGS (SEQ ID NO: 5) as a structural unit). The number of amino acid residues of the linker is, for example, 5 to 30, preferably 10 to 20, and more preferably 15. Arrangement of the heavy-chain variable region and the light-chain variable region is not limited. The light-chain variable region may be at a position closer to the N-terminus, or the heavy-chain variable region may be at a position closer to the N-terminus. The arrangement is preferably the former.

The antigen receptor of the present invention typically contains a core domain containing a variable region of an antibody or T-cell receptor (e.g., a scFv domain containing a heavy-chain variable region and a light-chain variable region), a transmembrane domain, and the intracellular domain of TCR. In the core domain, the variable region, the transmembrane domain, and the intracellular domain of TCR are arranged in this order from the N-terminus directly or via other domains.

The antigen receptor of the present invention containing the core domain preferably contains the GITRL domain at a position closer to the C-terminus of the core domain via a self-cleaving peptide domain.

The transmembrane domain can be of any type that does not interfere with the functionality of the antigen receptor. For example, CD28, CD3ζ, CD4, or CD8α, which are expressed in cells such as T cells, can be used. Of these, the transmembrane domain is preferably CD28. These transmembrane domains may be mutated as long as the functionality of the antigen receptor is not interfered with.

The intracellular domain of TCR can be, for example, an intracellular domain derived from CD3, which is also called a "TCRζ chain." CD3 may be mutated as long as the functionality of the antigen receptor is not interfered with. Mutation of CD3 is preferably made such that CD3 contains ITAM (immunoreceptor tyrosine-based activation motif).

The antigen receptor of the present invention preferably has a spacer sequence between the variable region and the transmembrane domain. The spacer sequence can be of any length and can be formed of any amino acid residues as long as the functionality of the antigen receptor is not interfered with. For example, the spacer sequence can be designed so as to have about 10 to 200 amino acid residues. The spacer sequence for use is preferably the sequence of the constant region of the light chain.

The core domain in the antigen receptor of the present invention preferably further contains the intracellular domain of a co-stimulator. The intracellular domain of a co-stimulator can be of any intracellular domain derived from a co-stimulator of cells such as T cells. For example, at least one member selected from the group consisting of OX40, 4-1BB, GITR, CD27, CD278, CD28 and the like can be suitably selected and used. Of these, CD28 is preferable from the standpoint of the notably excellent effect due to its combination with the GITRL domain. The intracellular domain of these co-stimulators may be mutated as long as the functionality of the antigen receptor is not interfered with. The position of the intracellular domain of a co-stimulator is not particularly limited as long as the intracellular domain is at a position closer to the C-terminus of the transmembrane domain; the intracellular domain may be at a position closer to the N-terminus or the C-terminus of the intracellular domain of TCR.

In a particularly preferable embodiment of the present invention, a variable region, a spacer sequence, a transmembrane domain, the intracellular domain of a co-stimulator, the intracellular domain of TCR, a self-cleaving peptide domain, and the GITRL domain are arranged in this order from the N-terminus directly or via other domains.

The antigen receptor of the present invention may contain a ligand domain such as the 4-1BBL domain or ICOSL domain at a position closer to the C-terminus of the core domain via a self-cleaving peptide domain.

The antigen receptor of the present invention may be a molecule formed of a single type of polypeptide or a molecule formed of a complex of two or more types of polypeptides. The antigen receptor of the present invention may also be a molecule formed of a polypeptide or of a complex of polypeptides, or a molecule formed of a polypeptide or complex of polypeptides to which another substance (e.g., a fluorescent substance, a radioactive substance, or an inorganic particle) is linked.

The antigen receptor of the present invention may be chemically modified. The polypeptide that constitutes the antigen receptor of the present invention may have a carboxyl group (-COOH), carboxylate (-COO⁻), amide (-CONH₂), or ester (-COOR) at the C-terminus. "R" in the ester is, for example, a C₁₋₆ alkyl group such as methyl, ethyl, n-propyl, isopropyl, or n-butyl; a C₃₋₈ cycloalkyl group such as cyclopentyl or cyclohexyl; a C₆₋₁₂ aryl group such as phenyl or α-naphthyl; a phenyl-C₁₋₂ alkyl group such as benzyl or phenethyl; a C₇₋₁₄ aralkyl group such as an α-naphthyl-C₁₋₂ alkyl group such as α-naphthyl methyl; or a pivaloyloxymethyl group. The polypeptide that constitutes the antigen receptor of the present invention may have an amidated or esterified carboxyl group (or carboxylate), which is not the carboxyl group at the C-terminus. The ester in this case may be, for example, the esters of the C-terminus described above. The polypeptide that constitutes the antigen receptor of the present invention further includes polypeptides having the amino group of the N-terminus amino acid residue protected by a protective group (e.g., a C₁₋₆ acyl group including a C₁₋₆ alkanoyl such as a formyl group and an acetyl group), polypeptides having the N-terminal glutamine residue pyroglutamated that can be formed due to cleavage in vivo; and polypeptides having a substituent (e.g., -OH, -SH, an amino group, an imidazole group, an indole group, and a guanidino group) on a side change of an amino acid in the molecule protected by an appropriate protective group (e.g., a C₁₋₆ acyl group including a C₁₋₆ alkanoyl group such as a formyl group and an acetyl group).

The antigen receptor of the present invention may have a protein or peptide (e.g., a known protein tag or signal sequence) added. Examples of protein tags include biotin, a His tag, a FLAG tag, a Halo tag, a MBP tag, a HA tag, a Myc tag, a V5 tag, a PA tag, and a fluorescent protein tag.

The antigen receptor of the present invention may be a pharmaceutically acceptable salt formed with an acid or base. The salt can be any pharmaceutically acceptable salt, and can be either an acid salt or a basic salt. Examples of acid salts include inorganic acid salts, such as hydrochloride, hydrobromide, sulfate, nitrate, and phosphate; organic acid salts, such as acetate, propionate, tartarate, fumarate, maleate, malate, citrate, methanesulfonate, and para-toluenesulfonate; and amino acid salts, such as aspartate, and glutamate. Examples of basic salts include alkali metal salts such as sodium salts and potassium salts; and alkaline-earth metal salts, such as calcium salts and magnesium salts.

The antigen receptor of the present invention may be in the form of a solvate. The solvent can be any pharmaceutically acceptable solvent, and may be, for example, water, ethanol, glycerol, or acetic acid.

The techniques for producing an antigen receptor and a CAR-T cell that expresses the antigen receptor are known. Antigen receptors and CAR-T cells can be produced in accordance with a known method or an equivalent method.

### 3. Polynucleotide

In an embodiment, the present invention relates to a polynucleotide encoding the antigen receptor of the present invention (which may be referred to as "the polynucleotide of the present invention" in the present specification). The polynucleotide of the present invention is described below.

The polynucleotide of the present invention may contain other sequences in addition to the coding sequence of the antigen receptor of the present invention. Preferably, the polynucleotide of the present invention expressibly contains the sequence of the antigen receptor of the present invention. Other sequences include promoter sequences, enhancer sequences, repressor sequences, insulator sequences, origins of replication, reporter protein (e.g., fluorescent proteins) coding sequences, and drug-resistant-gene-coding sequences. The polynucleotide of the present invention may be a linear polynucleotide or a cyclic polynucleotide (e.g., a vector). The vector can be a plasmid vector or a virus vector (e.g., an adenovirus or retrovirus). The vector can also be, for example, a vector for cloning or for expression. The vector for expression includes vectors for prokaryotic cells, such as *Escherichia coli,* or actinomycetes, and vectors for eukaryotic cells, such as yeast cells, insect cells, or mammal cells.

The polynucleotide of the present invention includes not only DNA and RNA but also known chemically modified DNA or RNA as described below. To prevent the degradation by hydrolases such as nucleases, the phosphate residue (phosphate) of each nucleotide can be substituted with, for example, a chemically modified phosphate residue such as phosphorothioate (PS), methylphosphonate, or phosphorodithionate. The hydroxyl group at position 2 of the ribose of each ribonucleotide may also be substituted with -OR (R represents, for example, CH3(2'-O-Me), CH₂CH₂OCH₃ (2'-O-MOE), CH₂CH₂NHC(NH)NH₂, CH₂CONHCH₃, or CH₂CH₂CN). Additionally, the base moiety (pyrimidine, purine) may be chemically modified, by, for example, introduction of a methyl group or a cationic functional group into positon 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl portion, for example, by biotin, an amino group, a lower alkyl amine group, or an acetyl group. The term "polynucleotide" includes not only natural nucleic acids but also BNA (bridged nucleic acid), LNA (locked nucleic acid), and PNA (peptide nucleic acid).

### 4. Cell

In an embodiment, the present invention relates to a cell comprising the polynucleotide of the present invention (which may be referred to as "the cell of the present invention" in the present specification). The cell of the present invention is described below.

The cells from which the cell of the present invention is derived are not particularly limited. For the purpose of using the cell of the present invention in the production of the antigen receptor of the present invention, for example, cells that can be used for protein expression (e.g., insect cells, eukaryotic cells, mammal cells) can be used as the origin cells.

The cell of the present invention is preferably a T cell (e.g., an αβ T cell or a γδ T cell) or an NK cell. These cells are preferably cells expressing the antigen receptor of the present invention. In a more specific embodiment of these cells of the present invention, the antigen receptor of the present invention is expressed on the cell membrane, and preferably expressed in such a state that the antigen-binding domain is exposed outside the cell membrane.

A T cell or the like expressing the antigen receptor recognizes the antigen in the antigen-binding domain, and then intracellularly transfers a recognition signal to activate a signal that induces cytotoxic activity. In conjunction with this, the cell mounts attacks against other cells or tissues expressing the antigen or exerts cytotoxic activity.

When a cell exhibiting such a function is a CTL, this cell is called a "chimeric antigen receptor T-cell" ("CAR-T cell"). Cells that have potential to exhibit cytotoxic activity, such as NK cells, can also exert cytotoxic activity when the antigen-binding domain binds to the antigen, as with the chimeric antigen receptor T-cell. Thus, a host cell comprising the polynucleotide encoding the antigen receptor (in particular, a host cell having cytotoxic activity) is useful as an active ingredient of pharmaceutical compositions.

Such T cells or the like are useful for treatment or prevention of cancer or the like because they specifically recognize cancer tissue (tumor tissue). The type of cancer is not particularly limited, and includes solid cancer and blood cancer. Examples of solid cancer include lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, rhabdomyosarcoma, and the like.

The cell of the present invention can be obtained by introducing the polynucleotide of the present invention into cells. If necessary, the cell containing the polynucleotide of the present invention may be concentrated, or may be concentrated using a specific marker (CD antigen, such as CD8) as an indicator.

### 5. Pharmaceutical Composition

In an embodiment, the present invention relates to a pharmaceutical composition comprising the T cell or NK cell of the present invention (which may be referred to as "the pharmaceutical composition of the present invention" in the present specification). The pharmaceutical composition of the present invention is described below.

The content of the cell in the pharmaceutical composition can be appropriately set in consideration of the type of target disease (e.g., solid cancer), desired therapeutic effects, administration method, treatment period, patient's age, patient's body weight, etc. The content of the cell in the pharmaceutical composition may be, for example, about 1 cell/mL to 10⁴ cells/mL.

The administration form of the pharmaceutical composition is not particularly limited as long as the desired effects are obtained. The pharmaceutical composition can be administered to mammals, including humans, by any of the following administration routes: oral administration and parenteral administration (e.g., intravenous injection, intramuscular injection, subcutaneous administration, rectal administration, dermal administration, and local administration). Since the active ingredient is a cell, the administration form is preferably parenteral administration, and more preferably intravenous injection. The dosage forms for oral administration and parenteral administration, and their production methods are well known to a person skilled in the art. The pharmaceutical composition can be produced according to a usual method by, for example, mixing the cell of the present invention with a pharmaceutically acceptable carrier etc.

Examples of dosage forms for parenteral administration include injection preparations (e.g., intravenous drip infusion, intravenous injection, intramuscular injection, subcutaneous injection, and endodermic injection), external preparations (e.g., ointments, cataplasms, and lotions), suppositories, inhalants, eye drops, ophthalmic ointments, nasal drops, ear drops, liposome agents, and the like. For example, an injection preparation can be prepared by dissolving or suspending an antibody or cells in distilled water for injection, and optionally adding a solubilizer, a buffer, a pH adjuster, an isotonizing agent, a soothing agent, a preservative, a stabilizer, etc. The pharmaceutical composition can also be used as a freeze-dried preparation prepared before use.

The pharmaceutical composition may further comprise other drugs effective for the treatment or prevention of diseases. The pharmaceutical composition can also contain components such as sterilants, antiphlogistics, cell activators, vitamins, and amino acids, if necessary.

As the carrier used for formulating the pharmaceutical composition, excipients, binders, disintegrators, lubricants, coloring agents, and flavoring agents that are generally used in this technical field can be used; and stabilizers, emulsifiers, absorption enhancers, surfactants, pH adjusters, antiseptics, antioxidants, extenders, moisturizers, surface activators, dispersants, buffers, preservatives, solubilizers, soothing agents, and the like can also optionally be used.

The type of disease treated or prevented using the pharmaceutical composition is not particularly limited as long as the treatment or prevention can be achieved. Examples of specific target diseases include tumors. The type of tumor is not particularly limited, and includes solid cancer and blood cancer. Examples of solid cancer include lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, rhabdomyosarcoma, and the like.

The administration target (test subject) of the pharmaceutical composition is, for example, an animal having a disease described above or an animal with a potential to develop such a disease. A "potential to develop such a disease" can be determined by a known diagnostic method. The animal is, for example, a mammal, and preferably a human.

The dose of the pharmaceutical composition can be determined by a clinical physician, taking into consideration various factors, such as administration route, the type of disease, the degree of symptoms, patient's age, sex, and body weight, severity of disease, pharmacological findings such as pharmacokinetics and toxicological characteristics, use or nonuse of drug delivery system, and whether the composition is administered as part of a combinational drug with other medicinal agents. When the active ingredient is the cell, the dose of the pharmaceutical composition can be about 10⁴ cells/kg (body weight) to 10⁹ cells/kg (body weight). The administration schedule of the pharmaceutical composition can also be determined taking into consideration the same factors as those for the dose. For example, the composition can be administered once a day to once a month in the daily dose described above.

### Examples

The present invention is described in detail below with reference to Examples. However, the present invention is not limited to these Examples.

### Test Example 1: Construction of CAR Construct

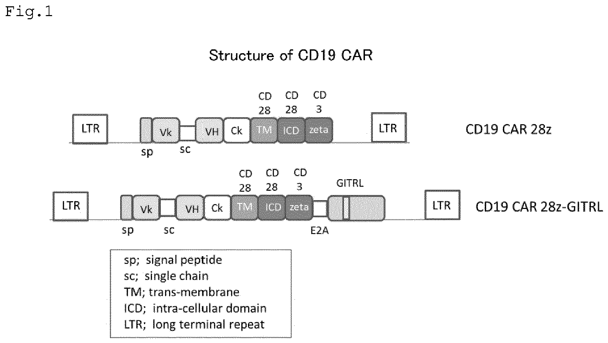

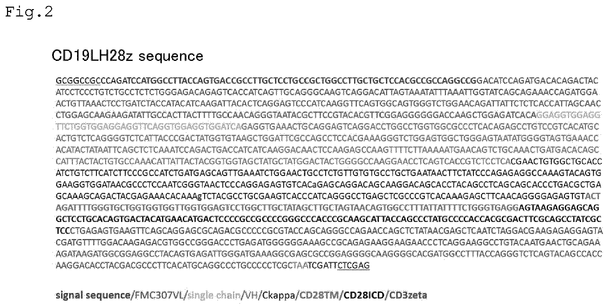

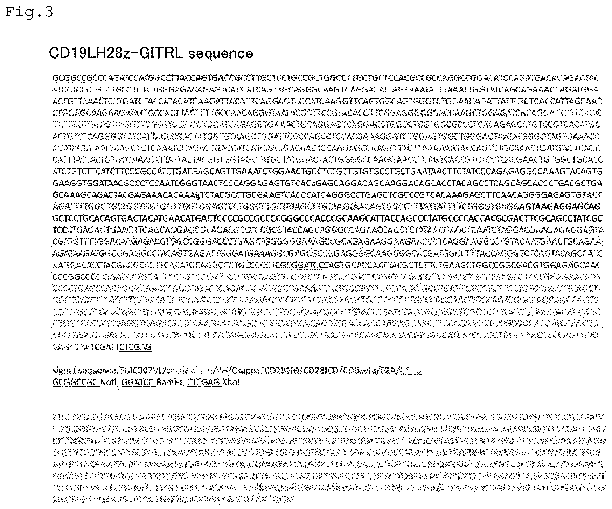

A CAR construct of Anti-CD19 (FMC-63)-28z or Anti-CD19 (FMC-63)-28z-GITRL was recombined to pMS3 to prepare a plasmid vector, and the plasmid vector was introduced into Plat-A cells (Cell Biolabs) with FuGENE (Promega) to prepare retroviruses. Fig. 1 shows the structures of CD19 28z CAR and CD19 28z GITRL CAR. Fig. 2 shows the base sequence (SEQ ID NO: 1) of CD19 28z CAR, and Fig. 3 shows the base sequence (SEQ ID NO: 2) and amino acid sequence (SEQ ID NO: 3) of CD19 28z GITRL CAR.

Retroviruses were also prepared using a CAR construct of Anti-CD19 (FMC-63)-zG or Anti-CD19 (FMC-63)-zG-GITRL. Specifically, retroviruses were prepared in the same manner, except that the region of the N-terminal side-CD28 intracellular domain-CD3ζ domain of CD19 28z CAR and CD19 28z GITRL CAR was replaced with the region of the N-terminal side-CD3ζ domain-GITR domain.

### Test Example 2: Making Effector Cell

αβ cells (obtained by culturing peripheral blood mononuclear cells isolated with Ficoll-Paque in GT-T551 supplemented with 0.6% inactivated human plasma and IL-2 at a final concentration of 600 IU/ml on a 12-well plate having 2 µg of OKT3 and 10 µg of RetroNectin immobilized thereon, and collecting the cells on day 4) and γδ cells (obtained by culturing peripheral blood mononuclear cells in YM-AB containing a novel bisphosphonate preparation (PTA), adding 25 ng/ml of IL-7 and 25 ng/ml of IL-15, and collecting the cells on day 4) were infected with the above retroviruses immobilized.

### Test Example 3: Confirmation of CAR expression

CAR expression was measured with a flow cytometer. After reaction with an anti-human κ antibody (rabbit IgG), the cells were reacted with Alexa 488-labeled anti-rabbit IgG (goat polyclonal) (Invitrogen). Further, for αβ cells, staining with APC/Cy7-labeled anti-human CD8 (BD) and an APC-labeled anti-human CD4 antibody (BioLegend) was added, and for γδ cells, staining with a PE-labeled anti-human Vδ2 antibody was added, followed by measuring with a FACSCanto. The expression rates of CAR molecules (recognized by anti-human κ antibody) in the CD4 and CD8 fractions of the αβ cells, and in the Vδ2-positive fraction of the γδ cells were calculated.

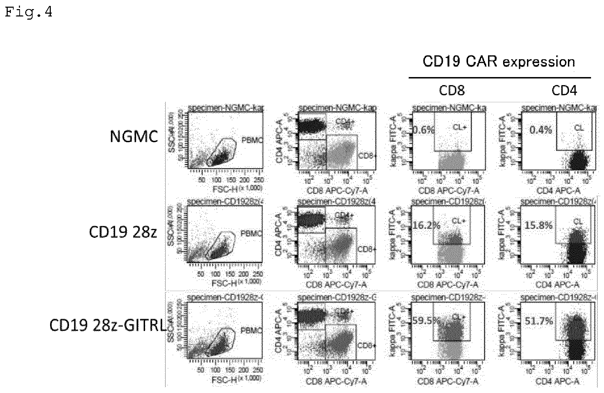

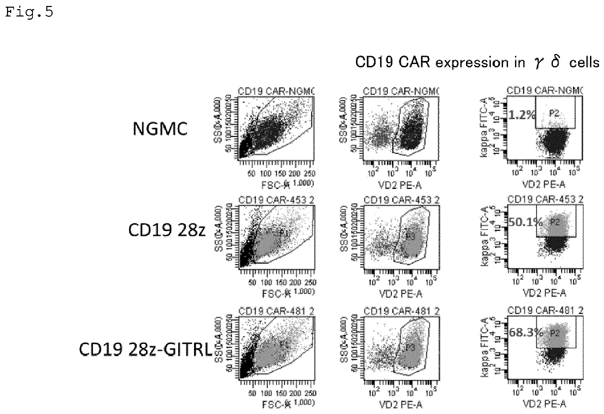

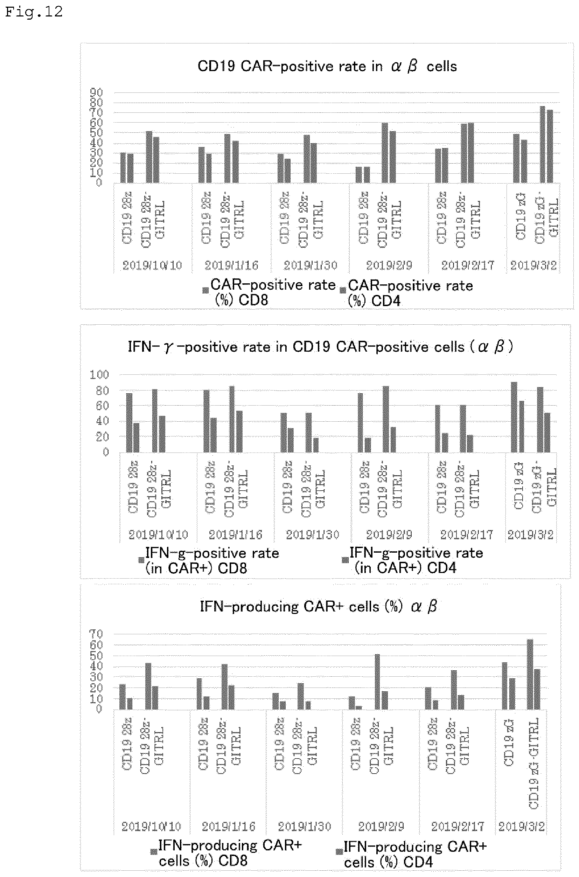

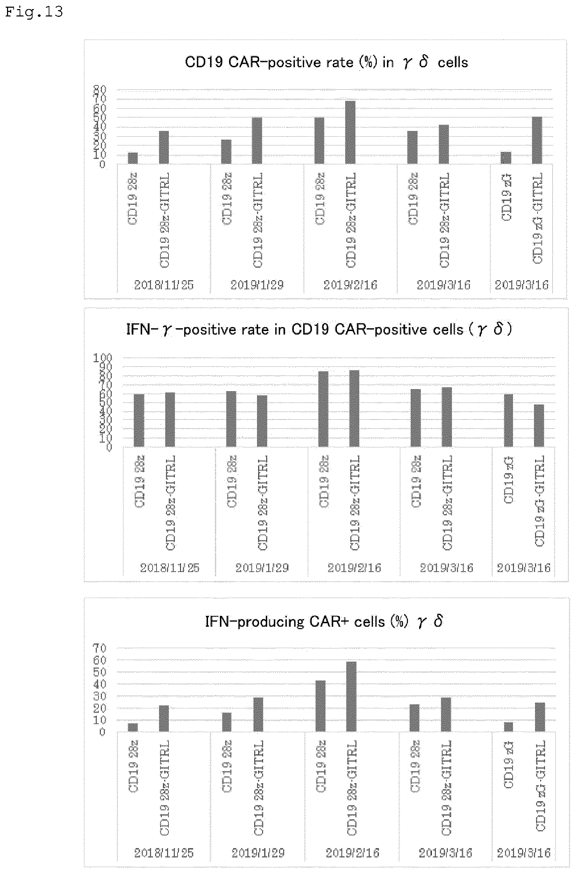

Regarding CD19 28z and CD19 28z-GITRL used for gene transfer, the expression of CD19 CAR was higher in cells in which CD19 28z-GITRL was introduced, in both the αβ cells (five gene transfer experiments) and the γδ cells (four gene transfer experiments) (Figs. 4 and 5, and the top row of each of Figs. 12 and 13).

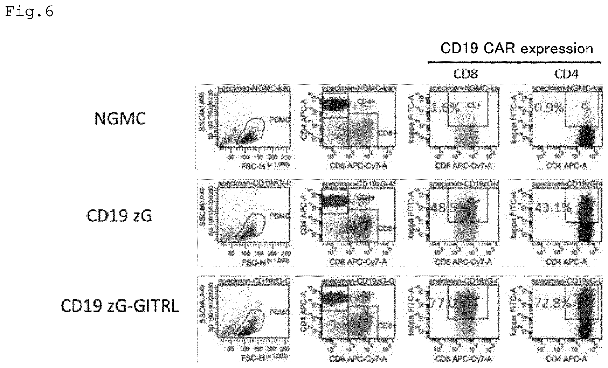

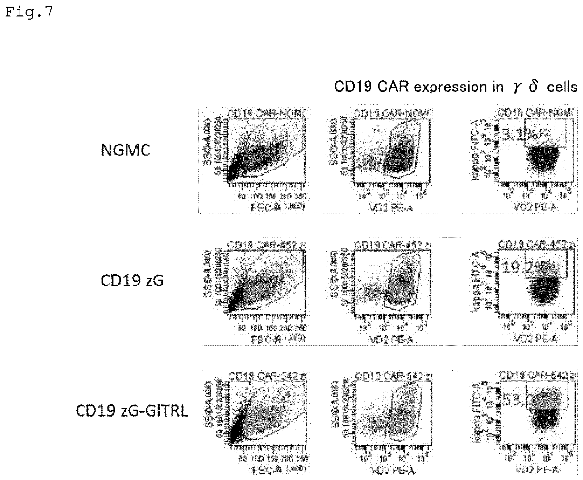

For each of CD19 zG and CD19 zG-GITRL, whose intracellular domain is zG, one gene transfer experiment was performed using αβ cells, and one gene transfer experiment was performed using γδ cells. As in the case of 28z, the expression of CD19 CAR was higher when the GITRL-bound form was used (Figs. 6 and 7, and the rightmost column in the top row of each of Figs. 12 and 13).

### Test Example 4: Recognition of Target Cell by Effector Cell

After addition of an APC-labeled anti-human CD107a antibody (BD), co-culture with CD19-positive B-acute lymphocytic leukemia cell line NALM6 (CD19-negative K562 was used as a control) was performed for 4 hours. Thereafter, the CAR-T cell surface antigen was stained in the same manner as above, and cytokines in the cells after treatment with Cytofix/Cytoperm (BD) were stained with V450-labeled anti-human IFN-γ and PE/Cy7-labeled anti-human TNF-α, followed by measurement with a FACSCanto II. The CD107a, IFN-γ, and TNF-α positive rates of the CAR-positive cells in the CD4 and CD8 fractions of the αβ cells and in the Vδ2-positive fraction of the γδ cells were calculated.

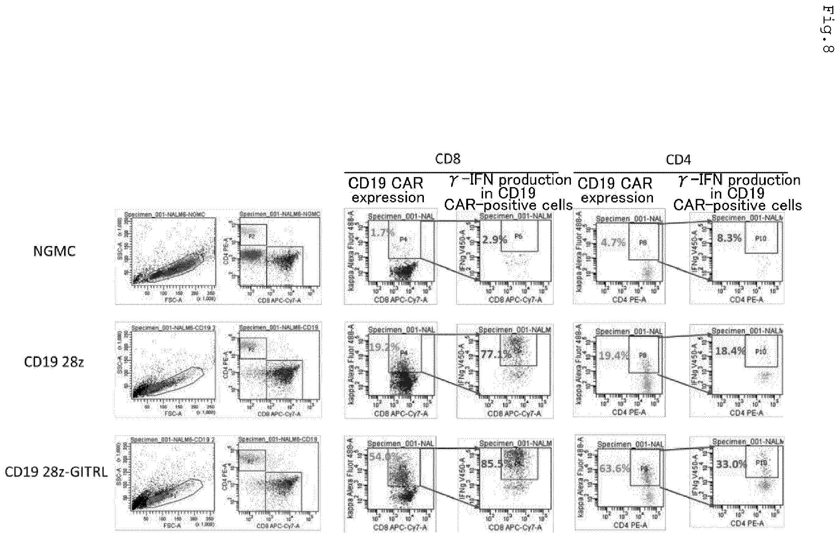

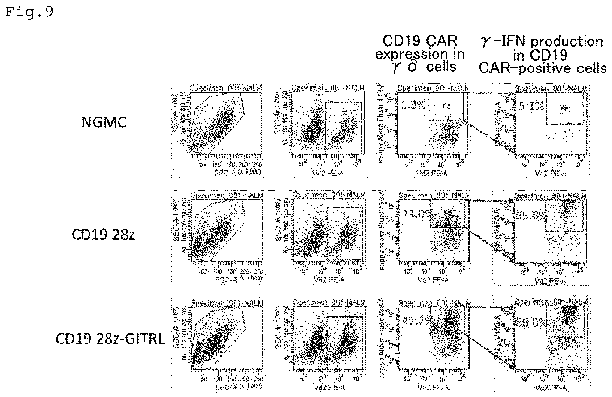

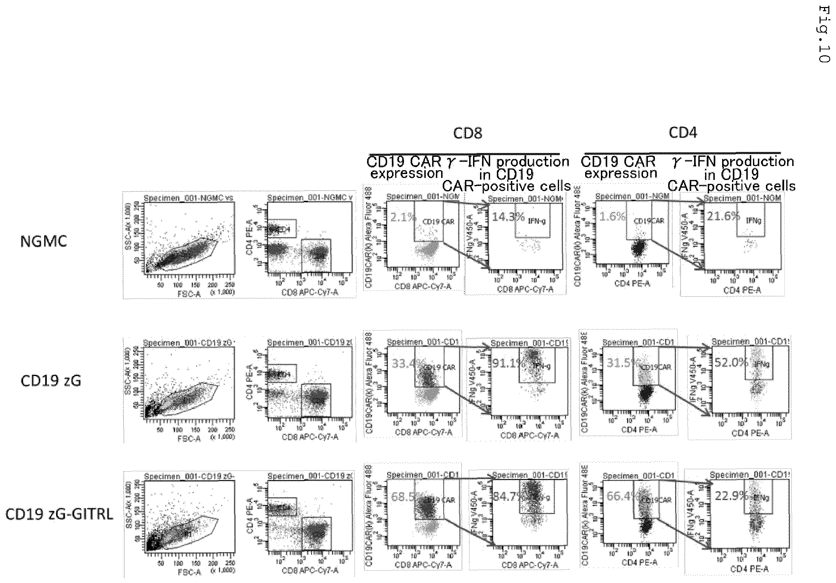

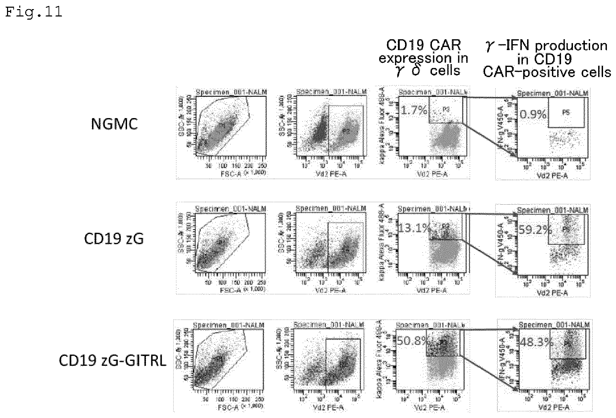

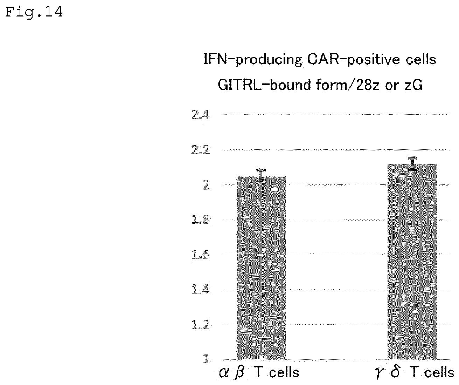

The proportion of IFN-γ-producing cells in the CD19CAR-positive cells after 4-hour co-culture with the target cell line (NALM6) tended to be higher in both the αβ cells (28z: Fig. 8, zG: Fig. 10) and the γδ cells (28z: Fig. 9, and zG: Fig. 11) when the GITRL-bound form was used, but there was no significant difference (middle row of each of Figs. 12 and 13). However, the number of IFN-γ-producing CD19CAR-positive cells was larger in the GITRL-bound form-transduced cells (both the αβ cells and the γδ cells) (the bottom row of each of Figs. 12 and 13). On average, the value in the GITRL-bound form-transduced cells was about double (Fig. 14).

### Test Example 5: Confirmation of Cytotoxic Action of Effector Cell

Cytotoxic activity over time was measured by real-time CTL assay (xCELLigence) by co-culture with NALM6. Specifically, an anti-CD9 antibody (ACEA Bioscience kit, catalog number: 8710247) adjusted to a final concentration of 4 µg/mL with tethering buffer (catalog number: 8718617) was immobilized on each well of an E-Plate 16. Three hours later, washing with PBS was performed, and target cells NALM6 (6.0×10⁴) suspended in 100 µl of a RPMI 1640 10% FCS-supplemented medium were added and allowed to stand at room temperature in a clean bench for 0.5 hours, followed by culture in an incubator under the conditions of 0.5% CO₂ and 37°C for 24 hours or more. Each kind of effector cells (6×10⁴) was added and allowed to stand at room temperature in a clean bench for 0.5 hours, followed by additional culture in an incubator under the conditions of CO₂ and 37°C for 5 to 7 days. Changes in electrical resistance over time were recorded in terms of cytotoxicity against NALM6 cells (xCELLigence method, ACEA Bioscience). As an effector, CD19 28zCAR-transduced αβ-T cells or γδ-T cells, or GITRL-co-expressing CD19 28zCAR-transduced αβ-T cells or γδ-T cells were used.

In the real-time CTL assay for 120 hours, both the CD19 28z-GITRL-transduced αβ cells and the CD19 28z-GITRL-transduced γδ cells showed more potent cytotoxic activity than the CD19 28z-transduce cells. Specifically, CD19 28z-GITRL was superior in terms of magnitude of cytotoxic activity, durability/persistence of maximum cytotoxic activity, and quickness, which is indicated by the time required to reach one half of the maximum cytotoxic activity (Figs. 15 and 16).

### Test Example 6: Investigation of Antitumor Effect

The antitumor effect on NALM6 cells transplanted into immunodeficient (NOG) mice was investigated. After NOG mice were subjected to 2 Gy irradiation, NALM6 (1×10⁶) into which the luciferase gene was introduced was transplanted and engrafted, each kind of CD19 CAR-T cells (5×10⁶) was infused. Images were taken by IVIS imaging over time to observe the kinetics of the NALM6 cells. Specifically, the experiment was performed as follows.

Immediately after 6-week-old female immunodeficient mice (NOD/Shi-scid/IL-2Rgamma^{null}) were subjected to 2Gy total body irradiation (TBI), CD19-positive leukemia cells into which the luciferase gene was introduced (NALM6/Luc) were injected to each mouse through the tail vein in an amount of 1×10⁶ cells/mouse (on day -7). One week later (on day 0), mice were injected through the tail vein with 200 µL/mouse of PBS (untreated group; two mice), and mice were injected through the tail vein with T lymphocytes from the same donor without CD19 CAR gene transfer (NGM-αβ T lymphocyte-treated group; 5×10⁶ cells/mouse; two mice) (both of these groups are negative controls); and a control group (three mice) was intravenously injected with conventional CD19-28z CAR-transduced αβ T lymphocytes, and a treatment group (three mice) was intravenously injected with the desired CD19 28z-GITRL CAR-transduced αβ T lymphocytes. The amount of remaining leukemia cells luminescent upon administration of luciferin was monitored over time using PerkinElmer's in vivo imaging system (IVIS).

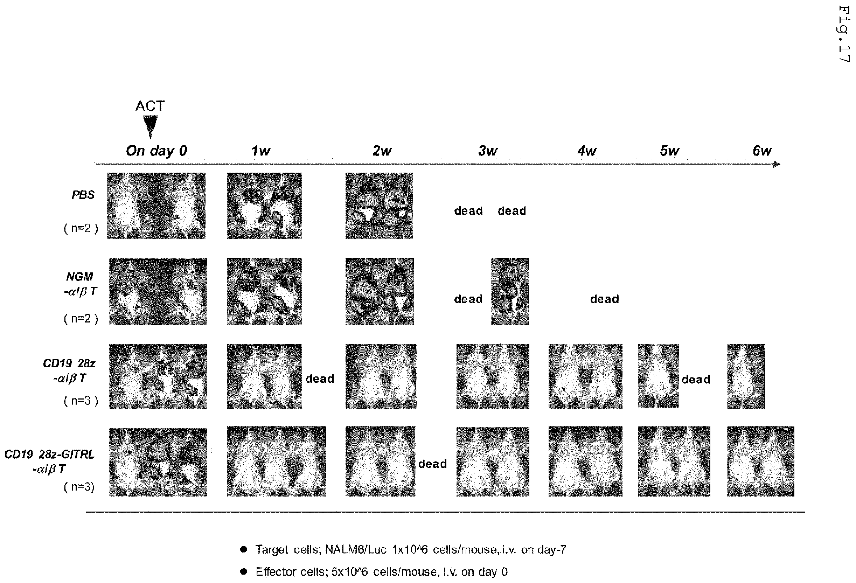

As shown in Fig. 17, the untreated group (PBS-treated group) died from tumor within 3 weeks, and the administration group of T lymphocytes that was not genetically modified (NGM-T administration group) also died from tumor within 4 weeks. In contrast, in both the 28z CAR-T administration group and the 28z-GITRL CAR-T administration group, the leukemia cells were eliminated 1 week after administration, and no relapse of leukemia was observed until 7 weeks after administration. However, the survival of the 28z-CAR-T-treated group tended to be shorter than that of the 28z-GITRL CAR-T-treated group.

### Test Example 7: Analysis of IFN-γ Production

After co-culture of CD19-positive leukemia cell line NALM6 (2×10⁵) with CD19 28z CAR-T cells or GITRL-co-expressing CD19 28z CAR-T cells (2×10⁵) for 4 hours, the NALM6-reactive IFN-γ production of each kind of the effector cells was measured with a flow cytometer (BD FACSCanto II).

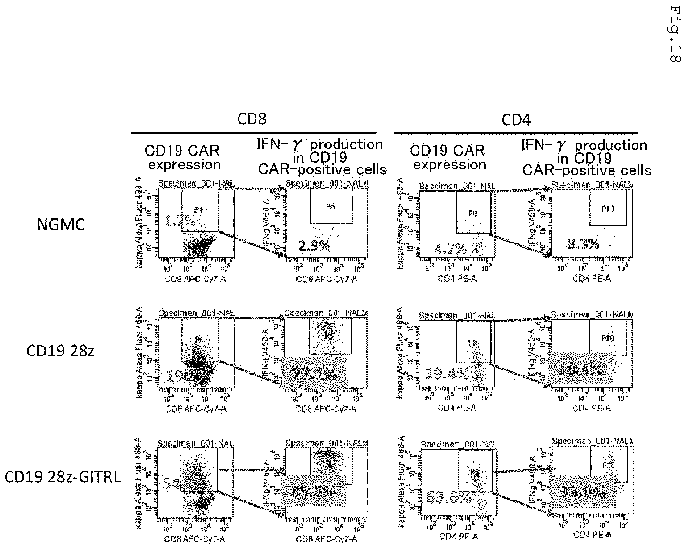

Fig. 18 shows the results. The GITRL-co-expressing CD19 CAR-T (αβ) cells showed increased IFN-γ production after co-culture with the target leukemia cells (NALM6).

### Test Example 8: Central Memory Analysis

Staining of CD8-positive αβ T cells into which CD19 28z CAR or GITRL-co-expressing CD19 28z CAR was introduced was performed with tetramethylrhodamine methyl ester (TMRM), which indicates mitochondrial membrane potential, in addition to a CD45RA antibody and a CD62L antibody, both of which indicate cell differentiation stages.

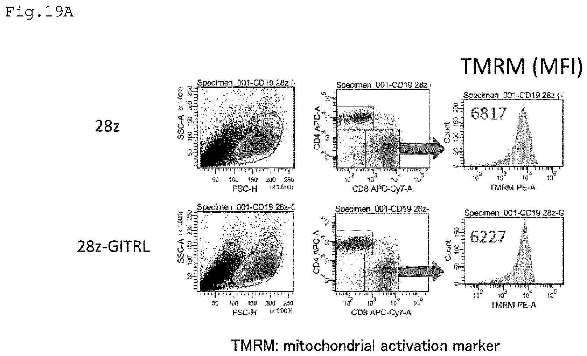

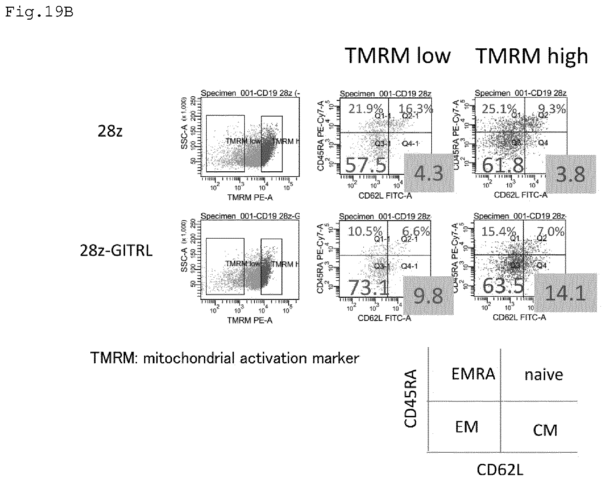

Fig. 19 shows the results. Compared with the 28z CAR-transduced CD8-positive T cells, an increase in an immunological memory cell group (CD62L-CD45RA-, effector memory T cells (T_{EM}) and CD62L+CD45RA-, central memory T cells (T_{CM}) ) was observed in the 28z-GITRL CAR-transduced CD8-positive T cell group. This tendency was more pronounced in T_{CM}. In addition, these immunological memory cells were confirmed to be immunological memory cells from their metabolic characteristics that were accompanied by an increase in mitochondrial membrane potential and depended on oxidative phosphorylation for their energy. These properties are considered to contribute to long-term engraftment of CAR-T cells infused into the patient's body, indicating the superiority of the GITRL-co-expressing CAR gene.

### Test Example 9: Analysis of Continuous Cytotoxic Activity

CEA-positive BxPC-3 cells (7000 cells) and CEA-negative MIA Paca-2 cells (7000 cells) were cultured on an E-plate, and effector cells (21000 cells) made from γδ cells were added thereto. Co-culture was performed, and the cytotoxicity for 24 hours was recorded over time. After completion of co-culture, the effector cells were collected and co-cultured with new target cells for 24 hours, and changes over time were recorded. The third co-culture was performed in the same manner, and changes over time were recorded.

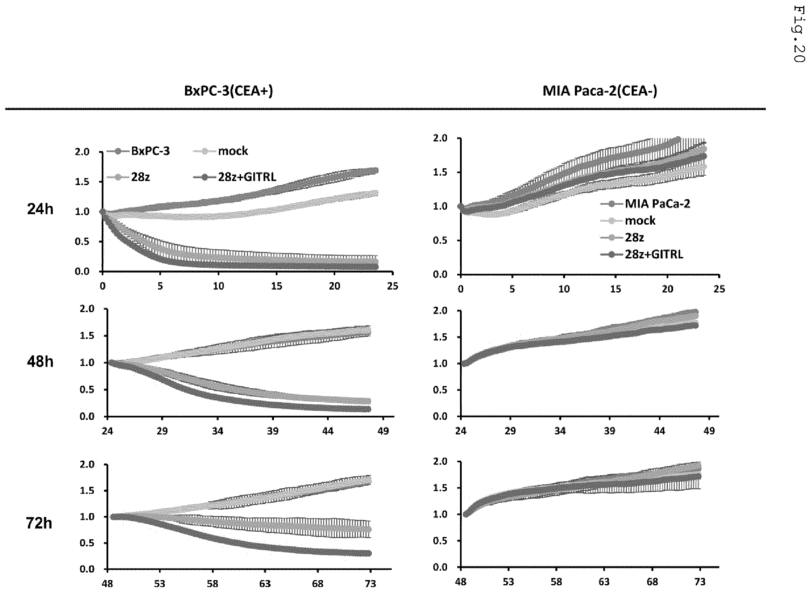

Fig. 20 shows the results. The cytotoxic activity was higher when the GITRL-co-expressing CEA 28z CAR-T cells were used than when the CEA 28z CAR-T cells were used. It was observed that the difference in cytotoxic activity tended to increase as the number of cultures performed increased.

### Test Example 10: Analysis 1 of Remaining Cell

CEA-positive BxPC-3 cells (5×10⁶) were subcutaneously transplanted into NOG mice to cause the mice to bear tumors. Thereafter, GITRL-co-expressing CEA 28z CAR-T cells and CEA 28z CAR-T cells, both of which were made from γδ cells, and mock cells into which no gene was introduced (each in an amount of 1×10⁷) were individually infused by intravenous injection. The abundance ratio of infused cells in peripheral blood after 2 weeks and 3 weeks was examined by anti-Vd2 antibody staining and biotinylated CEA staining. The number of mice used in the experiment was 2, 2, and 3.

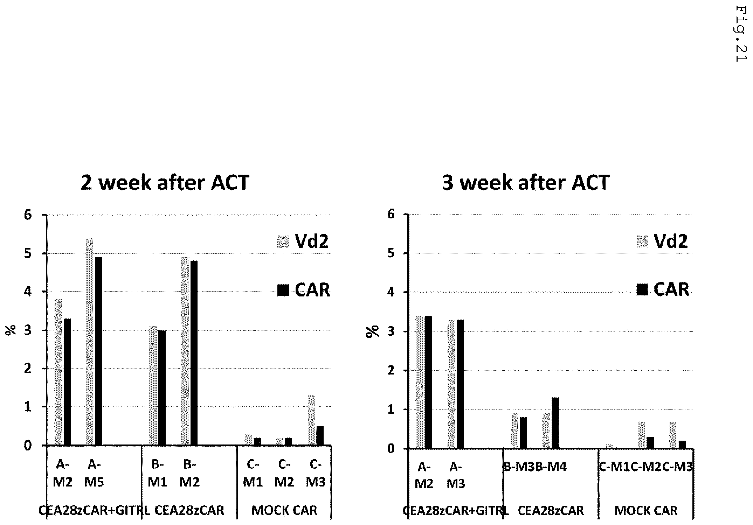

Fig. 21 shows the results. In the body of the NOG mice 3 weeks after infusion of the effector cells, the number of remaining cells was higher when the GITRL-co-expressing γδ CEA 28z CAR-T cells were infused than when the γδ CEA 28z CAR-T cells were infused.

### Test Example 11: Analysis 2 of Remaining Cell

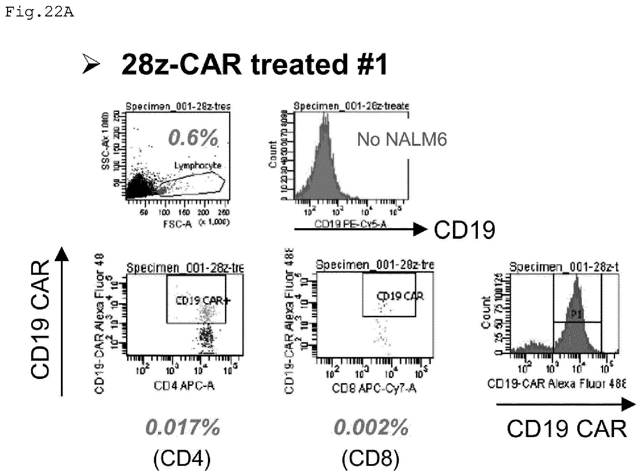

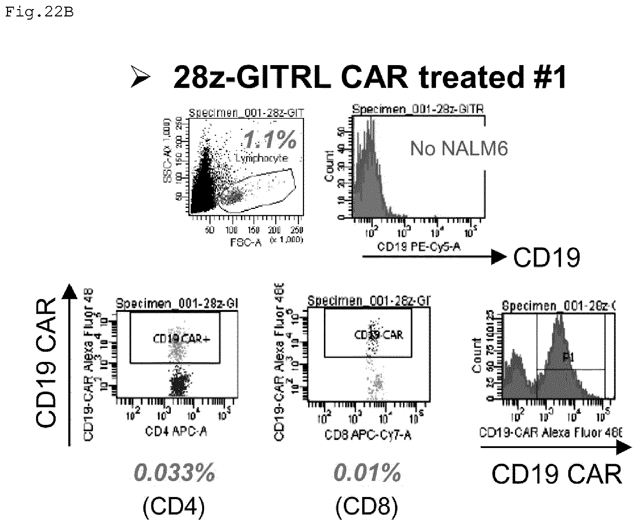

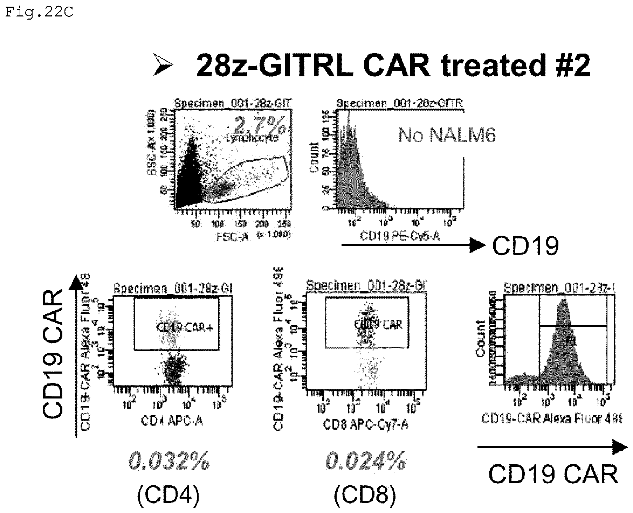

On day 7 after intravenous inoculation of luciferaselabeled leukemia cells NALM6 to NOG mice to cause the mice to bear tumors, CD19 28 CAR-T cells (αβ) or CD19 28z-GITRL CAR-T cells (αβ) were infused. The disappearance of the tumor cells was confirmed 14 days after infusion, and the same number of NALM6 cells were inoculated to cause the mice to bear tumors on day 28. On day 38, the venous blood of the mice was examined. Both the mice treated with the CD19 28 CAR-T cells and the mice treated with the CD19 28z-GITRL CAR-T cells again rejected NALM6, indicating that the anti-leukemia effect was sustained. Regarding the remaining infused CAR-T cells stained with an anti-kappa antibody, the amount of the remaining CD8+CAR+ cells was 0.002%, and the amount of the remaining CD4+CAR+ cells was 0.017% in the peripheral blood of the 28z CAR-T cell-infused mice, whereas in the two 28z-GITRL-CAR-T cell-infused mice, the amounts of the remaining CD8+CAR+ cells were 0.01% and 0.024%, and the amounts of the remaining CD4+CAR+ cells were 0.033% and 0.032% in the peripheral blood. Both CD8+CAR+ cells and CD4+CAR+ cells remained more abundant in the 28z-GITRL CAR-T cell-infused mice (Fig. 22).

### Test Example 12: Analysis of Engrafted Cell

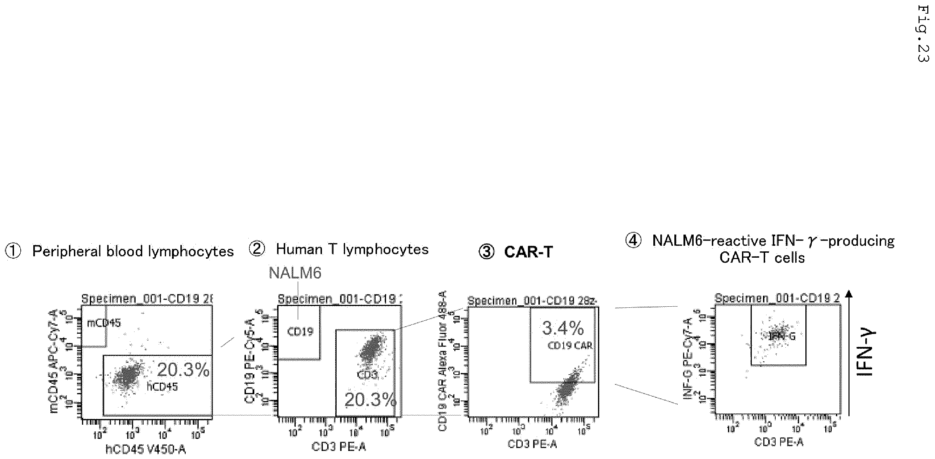

Peripheral blood was analyzed 55 days after cell therapy in mouse #2 treated with the 28z-GITLR CAR-T cells (αβ) in the NALM6 cell-bearing NOG mouse treatment model (mentioned above). In the mouse peripheral blood lymphocytes, 20.3% human lymphocytes were found (1 of Fig. 23). Human CD3-positive T lymphocytes were detected in 20.3% of the lymphocytes, and no CD19-positive human leukemia cells NALM6 were detected (2 of Fig. 23). CAR-T cells were found in 3.4% of the human T lymphocytes (3 of Fig. 23). When this mouse peripheral blood was co-cultured with NALM6 cells again in a test tube for 18 hours, all CAR-positive human lymphocytes retained their NALM6-reactive IFN-γ production capability (reactivity to leukemia cells) (4 of Fig. 23) .

### Conclusion

Compared with CD19 28z, CD19 28z-GITRL enables the CAR molecule to be expressed efficiently in both αβ and γδ T cells. Even when the intracellular domain is zG instead of 28z, the GITRL-bound form allows for high efficiency of CAR molecule expression. Moreover, although the superiority of the GITRL-bound form was not found in cytokine production by co-culture with target cells for a short period of time (4 hours), the GITRL-bound form showed potent, rapid, and long-lasting target-specific cytotoxic activity in both αβ and γδ T cells in the real-time CTL assay. From these results, the GITRL-bound CAR-T cells are considered to induce enhanced antitumor activity based on enhanced activation and prolonged duration in the T-cell activation process following antigen recognition, rather than the antigen recognition process. Thus, the GITRL-bound CAR-T cells are also expected to have a clinical effect on refractory and relapsed cases, which are problems in current CD19 CAR-T (28z) cell therapy.

Moreover, the same tendency as above was observed when GD2 was targeted.

## Claims

1. An antigen receptor comprising a GITRL domain at a position closer to the C-terminus via a self-cleaving peptide domain.

2. The antigen receptor according to claim 1, comprising a core domain containing a variable region of an antibody or T-cell receptor, a transmembrane domain, and an intracellular domain of TCR.

3. The antigen receptor according to claim 2, comprising the GITRL domain at a position closer to the C-terminus of the core domain via a self-cleaving peptide domain.

4. The antigen receptor according to claim 2 or 3, wherein the core domain further contains an intracellular domain of a co-stimulator.

5. The antigen receptor according to claim 4, wherein the co-stimulator is CD28.

6. The antigen receptor according to any one of claims 1 to 5, which is a chimeric antigen receptor or a T-cell receptor.

7. The antigen receptor according to any one of claims 2 to 6, wherein the variable region is capable of binding to CD19.

8. A polynucleotide encoding the antigen receptor of any one of claims 1 to 7.

9. A cell comprising the polynucleotide of claim 8.

10. The cell according to claim 9, which is a α/β T cell, a γ/δ T cell, or an NK cell.

11. A pharmaceutical composition comprising the cell of claim 10.

12. The pharmaceutical composition according to claim 11, which is for use in the treatment or prevention of cancer.


SEQUENCE LISTING

<110>  MIE UNIVERSITY

<120>  CAR or TCR

<130>  P20-149WO

<150>  JP 2019-142357
<151>  2019-08-01

<160>  5

<170>  PatentIn version 3.5

<210>  1
<211>  1693
<212>  DNA
<213>  Artificial Sequence

<220>
<223>  CD19 28z CAR base seq

<400>  1
gcggccgccc agatccatgg ccttaccagt gaccgccttg ctcctgccgc tggccttgct      60

gctccacgcc gccaggccgg acatccagat gacacagact acatcctccc tgtctgcctc     120

tctgggagac agagtcacca tcagttgcag ggcaagtcag gacattagta aatatttaaa     180

ttggtatcag cagaaaccag atggaactgt taaactcctg atctaccata catcaagatt     240

acactcagga gtcccatcaa ggttcagtgg cagtgggtct ggaacagatt attctctcac     300

cattagcaac ctggagcaag aagatattgc cacttacttt tgccaacagg gtaatacgct     360

tccgtacacg ttcggagggg ggaccaagct ggagatcaca ggaggtggag gttctggtgg     420

aggaggttca ggtggaggtg gatcagaggt gaaactgcag gagtcaggac ctggcctggt     480

ggcgccctca cagagcctgt ccgtcacatg cactgtctca ggggtctcat tacccgacta     540

tggtgtaagc tggattcgcc agcctccacg aaagggtctg gagtggctgg gagtaatatg     600

gggtagtgaa accacatact ataattcagc tctcaaatcc agactgacca tcatcaagga     660

caactccaag agccaagttt tcttaaaaat gaacagtctg caaactgatg acacagccat     720

ttactactgt gccaaacatt attactacgg tggtagctat gctatggact actggggcca     780

aggaacctca gtcaccgtct cctcacgaac tgtggctgca ccatctgtct tcatcttccc     840

gccatctgat gagcagttga aatctggaac tgcctctgtt gtgtgcctgc tgaataactt     900

ctatcccaga gaggccaaag tacagtggaa ggtggataac gccctccaat cgggtaactc     960

ccaggagagt gtcacagagc aggacagcaa ggacagcacc tacagcctca gcagcaccct    1020

gacgctgagc aaagcagact acgagaaaca caaagtctac gcctgcgaag tcacccatca    1080

gggcctgagc tcgcccgtca caaagagctt caacagggga gagtgtacta gattttgggt    1140

gctggtggtg gttggtggag tcctggcttg ctatagcttg ctagtaacag tggcctttat    1200

tattttctgg gtgaggagta agaggagcag gctcctgcac agtgactaca tgaacatgac 1260

tccccgccgc cccgggccca cccgcaagca ttaccagccc tatgccccac cacgcgactt 1320

cgcagcctat cgctccctga gagtgaagtt cagcaggagc gcagacgccc ccgcgtacca 1380

gcagggccag aaccagctct ataacgagct caatctagga cgaagagagg agtacgatgt 1440

tttggacaag agacgtggcc gggaccctga gatgggggga aagccgcaga gaaggaagaa 1500

ccctcaggaa ggcctgtaca atgaactgca gaaagataag atggcggagg cctacagtga 1560

gattgggatg aaaggcgagc gccggagggg caaggggcac gatggccttt accagggtct 1620

cagtacagcc accaaggaca cctacgacgc ccttcacatg caggccctgc cccctcgcta 1680

atcgattctc gag 1693

<210> 2
<211> 2356
<212> DNA
<213> Artificial Sequence

<220>
<223> CD19 28z GITRL CAR base seq

<400> 2
gcggccgccc agatccatgg ccttaccagt gaccgccttg ctcctgccgc tggccttgct 60

gctccacgcc gccaggccgg acatccagat gacacagact acatcctccc tgtctgcctc 120

tctgggagac agagtcacca tcagttgcag ggcaagtcag gacattagta aatatttaaa 180

ttggtatcag cagaaaccag atggaactgt taaactcctg atctaccata catcaagatt 240

acactcagga gtcccatcaa ggttcagtgg cagtgggtct ggaacagatt attctctcac 300

cattagcaac ctggagcaag aagatattgc cacttacttt tgccaacagg gtaatacgct 360

tccgtacacg ttcggagggg ggaccaagct ggagatcaca ggaggtggag gttctggtgg 420

aggaggttca ggtggaggtg gatcagaggt gaaactgcag gagtcaggac ctggcctggt 480

ggcgccctca cagagcctgt ccgtcacatg cactgtctca ggggtctcat tacccgacta 540

tggtgtaagc tggattcgcc agcctccacg aaagggtctg gagtggctgg gagtaatatg 600

gggtagtgaa accacatact ataattcagc tctcaaatcc agactgacca tcatcaagga 660

caactccaag agccaagttt tcttaaaaat gaacagtctg caaactgatg acacagccat 720

ttactactgt gccaaacatt attactacgg tggtagctat gctatggact actggggcca 780

aggaacctca gtcaccgtct cctcacgaac tgtggctgca ccatctgtct tcatcttccc 840

gccatctgat gagcagttga aatctggaac tgcctctgtt gtgtgcctgc tgaataactt 900

ctatcccaga gaggccaaag tacagtggaa ggtggataac gccctccaat cgggtaactc 960

ccaggagagt gtcacagagc aggacagcaa ggacagcacc tacagcctca gcagcaccct 1020

gacgctgagc aaagcagact acgagaaaca caaagtctac gcctgcgaag tcacccatca 1080

gggcctgagc tcgcccgtca caaagagctt caacagggga gagtgtacta gattttgggt 1140

gctggtggtg gttggtggag tcctggcttg ctatagcttg ctagtaacag tggcctttat 1200

tattttctgg gtgaggagta agaggagcag gctcctgcac agtgactaca tgaacatgac 1260

tccccgccgc cccgggccca cccgcaagca ttaccagccc tatgccccac cacgcgactt 1320

cgcagcctat cgctccctga gagtgaagtt cagcaggagc gcagacgccc ccgcgtacca 1380

gcagggccag aaccagctct ataacgagct caatctagga cgaagagagg agtacgatgt 1440

tttggacaag agacgtggcc gggaccctga gatgggggga aagccgcaga gaaggaagaa 1500

ccctcaggaa ggcctgtaca atgaactgca gaaagataag atggcggagg cctacagtga 1560

gattgggatg aaaggcgagc gccggagggg caaggggcac gatggccttt accagggtct 1620

cagtacagcc accaaggaca cctacgacgc ccttcacatg caggccctgc cccctcgcgg 1680

atcccagtgc accaattacg ctcttctgaa gctggccggc gacgtggaga gcaaccccgg 1740

ccccatgacc ctgcacccca gccccatcac ctgcgagttc ctgttcagca ccgccctgat 1800

cagccccaag atgtgcctga gccacctgga gaacatgccc ctgagccaca gcagaaccca 1860

gggcgcccag agaagcagct ggaagctgtg gctgttctgc agcatcgtga tgctgctgtt 1920

cctgtgcagc ttcagctggc tgatcttcat cttcctgcag ctggagaccg ccaaggagcc 1980

ctgcatggcc aagttcggcc ccctgcccag caagtggcag atggccagca gcgagccccc 2040

ctgcgtgaac aaggtgagcg actggaagct ggagatcctg cagaacggcc tgtacctgat 2100

ctacggccag gtggccccca acgccaacta caacgacgtg gccccctteg aggtgagact 2160

gtacaagaac aaggacatga tccagaccct gaccaacaag agcaagatcc agaacgtggg 2220

cggcacctac gagctgcacg tgggcgacac catcgacctg atcttcaaca gcgagcacca 2280

ggtgctgaag aacaacacct actggggcat catcctgctg gccaaccccc agttcatcag 2340

ctaatcgatt ctcgag 2356

<210> 3
<211> 775
<212> PRT
<213> Artificial Sequence

<220>
<223> CD19 28z GITRL CAR aa seq

<400> 3

Met Ala Leu Pro Val Thr Ala Leu Leu Leu Pro Leu Ala Leu Leu Leu
1               5                   10                  15

His Ala Ala Arg Pro Asp Ile Gln Met Thr Gln Thr Thr Ser Ser Leu
            20                  25                  30

Ser Ala Ser Leu Gly Asp Arg Val Thr Ile Ser Cys Arg Ala Ser Gln
        35                  40                  45

Asp Ile Ser Lys Tyr Leu Asn Trp Tyr Gln Gln Lys Pro Asp Gly Thr
    50                  55                  60

Val Lys Leu Leu Ile Tyr His Thr Ser Arg Leu His Ser Gly Val Pro
65                  70                  75                  80

Ser Arg Phe Ser Gly Ser Gly Ser Gly Thr Asp Tyr Ser Leu Thr Ile
                85                  90                  95

Ser Asn Leu Glu Gln Glu Asp Ile Ala Thr Tyr Phe Cys Gln Gln Gly
            100                 105                 110

Asn Thr Leu Pro Tyr Thr Phe Gly Gly Gly Thr Lys Leu Glu Ile Thr
        115                 120                 125

Gly Gly Gly Gly Ser Gly Gly Gly Gly Ser Gly Gly Gly Gly Ser Glu
    130                 135                 140

Val Lys Leu Gln Glu Ser Gly Pro Gly Leu Val Ala Pro Ser Gln Ser
145                 150                 155                 160

Leu Ser Val Thr Cys Thr Val Ser Gly Val Ser Leu Pro Asp Tyr Gly
                165                 170                 175

Val Ser Trp Ile Arg Gln Pro Pro Arg Lys Gly Leu Glu Trp Leu Gly
            180                 185                 190

Val Ile Trp Gly Ser Glu Thr Thr Tyr Tyr Asn Ser Ala Leu Lys Ser
        195                 200                 205

Arg Leu Thr Ile Ile Lys Asp Asn Ser Lys Ser Gln Val Phe Leu Lys
    210                 215                 220

Met Asn Ser Leu Gln Thr Asp Asp Thr Ala Ile Tyr Tyr Cys Ala Lys
225                 230                 235                 240

His Tyr Tyr Tyr Gly Gly Ser Tyr Ala Met Asp Tyr Trp Gly Gln Gly
                245                 250                 255

Thr Ser Val Thr Val Ser Ser Arg Thr Val Ala Ala Pro Ser Val Phe
            260                 265                 270

Ile Phe Pro Pro Ser Asp Glu Gln Leu Lys Ser Gly Thr Ala Ser Val

275                 280                 285

Val Cys Leu Leu Asn Asn Phe Tyr Pro Arg Glu Ala Lys Val Gln Trp
    290                 295                 300

Lys Val Asp Asn Ala Leu Gln Ser Gly Asn Ser Gln Glu Ser Val Thr
305                 310                 315                 320

Glu Gln Asp Ser Lys Asp Ser Thr Tyr Ser Leu Ser Ser Thr Leu Thr
                325                 330                 335

Leu Ser Lys Ala Asp Tyr Glu Lys His Lys Val Tyr Ala Cys Glu Val
            340                 345                 350

Thr His Gln Gly Leu Ser Ser Pro Val Thr Lys Ser Phe Asn Arg Gly
        355                 360                 365

Glu Cys Thr Arg Phe Trp Val Leu Val Val Val Gly Gly Val Leu Ala
    370                 375                 380

Cys Tyr Ser Leu Leu Val Thr Val Ala Phe Ile Ile Phe Trp Val Arg
385                 390                 395                 400

Ser Lys Arg Ser Arg Leu Leu His Ser Asp Tyr Met Asn Met Thr Pro
                405                 410                 415

Arg Arg Pro Gly Pro Thr Arg Lys His Tyr Gln Pro Tyr Ala Pro Pro
            420                 425                 430

Arg Asp Phe Ala Ala Tyr Arg Ser Leu Arg Val Lys Phe Ser Arg Ser
        435                 440                 445

Ala Asp Ala Pro Ala Tyr Gln Gln Gly Gln Asn Gln Leu Tyr Asn Glu
    450                 455                 460

Leu Asn Leu Gly Arg Arg Glu Glu Tyr Asp Val Leu Asp Lys Arg Arg
465                 470                 475                 480

Gly Arg Asp Pro Glu Met Gly Gly Lys Pro Gln Arg Arg Lys Asn Pro
                485                 490                 495

Gln Glu Gly Leu Tyr Asn Glu Leu Gln Lys Asp Lys Met Ala Glu Ala
            500                 505                 510

Tyr Ser Glu Ile Gly Met Lys Gly Glu Arg Arg Arg Gly Lys Gly His
        515                 520                 525

Asp Gly Leu Tyr Gln Gly Leu Ser Thr Ala Thr Lys Asp Thr Tyr Asp
        530                 535                 540

Ala Leu His Met Gln Ala Leu Pro Pro Arg Gly Ser Gln Cys Thr Asn
545                 550                 555                 560

Tyr Ala Leu Leu Lys Leu Ala Gly Asp Val Glu Ser Asn Pro Gly Pro
                565                 570                 575

Met Thr Leu His Pro Ser Pro Ile Thr Cys Glu Phe Leu Phe Ser Thr
            580                 585                 590

Ala Leu Ile Ser Pro Lys Met Cys Leu Ser His Leu Glu Asn Met Pro
        595                 600                 605

Leu Ser His Ser Arg Thr Gln Gly Ala Gln Arg Ser Ser Trp Lys Leu
    610                 615                 620

Trp Leu Phe Cys Ser Ile Val Met Leu Leu Phe Leu Cys Ser Phe Ser
625                 630                 635                 640

Trp Leu Ile Phe Ile Phe Leu Gln Leu Glu Thr Ala Lys Glu Pro Cys
                645                 650                 655

Met Ala Lys Phe Gly Pro Leu Pro Ser Lys Trp Gln Met Ala Ser Ser
            660                 665                 670

Glu Pro Pro Cys Val Asn Lys Val Ser Asp Trp Lys Leu Glu Ile Leu
        675                 680                 685

Gln Asn Gly Leu Tyr Leu Ile Tyr Gly Gln Val Ala Pro Asn Ala Asn
    690                 695                 700

Tyr Asn Asp Val Ala Pro Phe Glu Val Arg Leu Tyr Lys Asn Lys Asp
705                 710                 715                 720

Met Ile Gln Thr Leu Thr Asn Lys Ser Lys Ile Gln Asn Val Gly Gly
                725                 730                 735

Thr Tyr Glu Leu His Val Gly Asp Thr Ile Asp Leu Ile Phe Asn Ser
            740                 745                 750

Glu His Gln Val Leu Lys Asn Asn Thr Tyr Trp Gly Ile Ile Leu Leu
        755                 760                 765

Ala Asn Pro Gln Phe Ile Ser
    770                 775

<210>  4
<211>  199
<212>  PRT
<213>  Homo sapiens

<400>  4

Met Thr Leu His Pro Ser Pro Ile Thr Cys Glu Phe Leu Phe Ser Thr
1               5                   10                  15

Ala Leu Ile Ser Pro Lys Met Cys Leu Ser His Leu Glu Asn Met Pro
            20                  25                  30

Leu Ser His Ser Arg Thr Gln Gly Ala Gln Arg Ser Ser Trp Lys Leu
        35                  40                  45

Trp Leu Phe Cys Ser Ile Val Met Leu Leu Phe Leu Cys Ser Phe Ser
    50                  55                  60

Trp Leu Ile Phe Ile Phe Leu Gln Leu Glu Thr Ala Lys Glu Pro Cys
65                  70                  75                  80

Met Ala Lys Phe Gly Pro Leu Pro Ser Lys Trp Gln Met Ala Ser Ser
                85                  90                  95

Glu Pro Pro Cys Val Asn Lys Val Ser Asp Trp Lys Leu Glu Ile Leu
            100                 105                 110

Gln Asn Gly Leu Tyr Leu Ile Tyr Gly Gln Val Ala Pro Asn Ala Asn
        115                 120                 125

Tyr Asn Asp Val Ala Pro Phe Glu Val Arg Leu Tyr Lys Asn Lys Asp
    130                 135                 140

Met Ile Gln Thr Leu Thr Asn Lys Ser Lys Ile Gln Asn Val Gly Gly
145                 150                 155                 160

Thr Tyr Glu Leu His Val Gly Asp Thr Ile Asp Leu Ile Phe Asn Ser
                165                 170                 175

Glu His Gln Val Leu Lys Asn Asn Thr Tyr Trp Gly Ile Ile Leu Leu
            180                 185                 190

Ala Asn Pro Gln Phe Ile Ser
        195


<210>  5
<211>  5
<212>  PRT

<213>  Artificial Sequence

<220>
<223>  GS linker

<400>  5

Gly Gly Gly Gly Ser
1               5